# FASCICULE DE BREVET EUROPEEN

(11) **EP 3 856 707 B1**
(45) Date de publication et mention de la délivrance du brevet: **10.08.2022**
(21) Numéro de dépôt: 19791316.3
(22) Date de dépôt: 09.09.2019
(51) Int. Cl.: C07C 17/42, C07C 21/18, F01K 25/10

(54) **STABILISATION DU 1-CHLORO-3,3,3-TRIFLUOROPROPENE**
STABILISIERUNG VON 1-CHLORO-3,3,3-TRIFLUORPROPEN
STABILIZATION OF 1-CHLORO-3,3,3-TRIFLUOROPROPENE

(30) Priorité: 26.09.2018 FR 1858817
(43) Date de publication de la demande: 04.08.2021
(73) Titulaire: ARKEMA FRANCE, 92700 Colombes (FR)
(72) Inventeur: BOUTIER, Jean-Christophe, 69493 PIERRE-BENITE Cedex (FR); RACHED, Wissam, 92705 COLOMBES cedex (FR)
(74) Mandataire: Arkema Patent
(86) Numéro de dépôt international: PCT/FR2019/052074
(87) Numéro de publication internationale: WO 2020/065166

(56) Documents cités:
- WO-A1-2016/146940
- WO-A1-2017/031046

## Description

### DOMAINE DE L'INVENTION

La présente invention concerne des composés permettant de stabiliser le 1-chloro-3,3,3-trifluoropropène et plus précisément de limiter ou d'empêcher l'isomérisation de la forme trans en forme cis, ou la dégradation du composé. L'invention concerne également l'utilisation de tels composés dans des applications de transfert de chaleur.

### ARRIERE-PLAN TECHNIQUE

Le trans-1-chloro-3,3,3-trifluoropropène (HCFO-1233zdE) est un produit présentant un faible potentiel de réchauffement climatique (GWP). Il possède des propriétés thermodynamiques et thermophysiques très favorables pour une utilisation comme fluide de transfert de chaleur dans les applications de refroidissement, de climatisation, de production d'électricité (notamment au moyen de cycles organiques de Rankine) et de pompes à chaleur à haute température.

Le HCFO-1233zdE présente une instabilité qui se manifeste surtout à température relativement élevée. Cette instabilité consiste en une isomérisation d'une fraction de la charge initiale aboutissant à la formation de cis-1-chloro-3,3,3-trifluoropropène (HCFO-1233zdZ).

Or, le HCFO-1233zdZ est un produit moins volatile que le HCFO-1233zdE. La température d'ébullition est de l'ordre de 40°C pour l'isomère Z, et de l'ordre de 18,3°C pour l'isomèreE. Cette différence implique un changement des propriétés thermodynamiques et thermophysiques du produit dans les installations, et une perte de performance, lorsque l'isomérisation se produit.

Le document WO 2016/146940 décrit l'utilisation de composés alcènes en C3-C6 pour la stabilisation du 1-chloro-3,3,3-trifluoropropène.

Le document FR 3 041 632 décrit un procède de purification et de séchage d'un flux d'hydrofluorooléfines comprenant une hydrofluorooléfine choisie parmi le HFO-1234yf et le HCFO-1233zd, de l'eau et des impuretés à base de composés carbonés halogénés, caractérisé en ce que ledit flux est mis en contact avec un agent adsorbant.

Le document FR 2 973 809 concerne l'utilisation de zéolithes afin d'améliorer la stabilité thermique de tout type d'huile et en particulier des huiles ou des formulations à base d'huiles entrant dans la composition de fluides réfrigérants.

Le document FR 2 973 717 concerne un procédé de réduction d'acidité des fluides frigorigènes, notamment de fluides frigorigènes utilisés dans les appareils de réfrigération et les climatiseurs, le procédé comprenant une étape de mise en contact du fluide frigorigène avec au moins un adsorbant zéolithique.

Le document FR 3 032 131 décrit l'utilisation, pour la séparation et/ou le séchage de gaz (notamment d'hydrofluorocarbures ou d'hydrofluorooléfines), de matériaux adsorbants zéolithiques particuliers.

Le document FR 3 041 632 concerne un procédé de purification et de séchage d'un flux d'hydrofluorooléfine comprenant une hydrofluorooléfine, de l'eau et des impuretés à base de composés carbonés halogénés, caractérisé en ce que ledit flux est mis en contact avec un agent adsorbant (notamment tamis moléculaire).

Le document WO 2012/067980 concerne un procédé de fabrication du HFO-1234yf par déshydrohalogénation d'un flux de 2-chloro-1,1,1,2-tétrafluoropropane qui ne contient pas des impuretés telles que des propanes, propènes et propynes halogénés.

Le document WO 2017/031046 décrit une méthode pour éliminer des impuretés acides présentes dans les oléfines halogénées telles que le HFO-1234ze, le HFO-1234yf, le HCFO-1233zd et le HCFO-1233xf, la méthode comprenant le passage du flux d'oléfine à travers un adsorbant solide qui peut notamment être un tamis moléculaire.

Le document EP 2 035 117 concerne un procédé de séchage d'un fluide comprenant un fluoropropène, le procédé comprenant une étape de mise en contact du fluide avec un déshydratant comprenant un tamis moléculaire.

Il existe un besoin de fournir une méthode améliorée permettant de limiter ou d'empêcher l'isomérisation du HCFO-1233zdE en HCFO-1233zdZ, notamment dans des systèmes de compression de vapeur tels que des systèmes de climatisation, de réfrigération, de pompe à chaleur et de cycle organique de Rankine, et tout particulièrement les systèmes comportant un évaporateur noyé.

### RESUME DE L'INVENTION

L'invention concerne en premier lieu l'utilisation conjointe d'un composé alcène en C3 à C6 et comportant une seule double liaison et d'au moins un tamis moléculaire pour limiter ou empêcher l'isomérisation du trans-1-chloro-3,3,3-trifluoropropène en cis-1-chloro-3,3,3-trifluoropropène, et/ou pour limiter ou empêcher la dégradation du trans-1-chloro-3,3,3-trifluoropropène.

Dans certains modes de réalisation, le composé alcène est un butène ou un pentène.

Dans certains modes de réalisation, le composé alcène présente :
- une température d'ébullition inférieure ou égale à 100°C, de préférence inférieure ou égale à 75°C, et de maniére plus particulièrement préférée inférieure ou égale à 50°C ; et/ou
- une température de solidification inférieure ou égale à 0°C, de préférence inférieure ou égale à -25°C, et de manière plus particulièrement préférée inférieure ou égale à -50°C.

Dans certains modes de réalisation, le composé alcène est le 2-méthyl-but-2-ène.

Dans certains modes de réalisation, le au moins un tamis moléculaire est au moins un adsorbant zéolithique.

Dans certains modes de réalisation, le au moins un adsorbant zéolithique est choisi parmi les zéolithes de type zéolithe A, les zéolithes de type faujasite, les zéolithes de type zéolithe Y, ainsi que les mélanges de celles-ci.

Dans certains modes de réalisation, le au moins un adsorbant zéolithique est choisi parmi la zéolithe 3A, la zéolithe 5A, la zéolithe 13X et les mélanges de celles-ci.

Dans certains modes de réalisation, on utilise au moins un tamis moléculaire pour adsorber de l'air et au moins un tamis moléculaire pour adsorber de l'eau, de préférence disposés en couches successives dans une cartouche.

L'invention concerne également un procédé de chauffage ou de refroidissement d'un fluide ou d'un corps au moyen d'un circuit de compression de vapeur contenant un fluide de transfert de chaleur, ledit procédé comprenant successivement : l'évaporation du fluide de transfert de chaleur, la compression du fluide de transfert de chaleur, la condensation du fluide de transfert de chaleur et la détente du fluide de transfert de chaleur, dans lequel le fluide de transfert de chaleur comprend du trans-1-chloro-3,3,3-trifluoropropène et un composé alcène en C3 à C6, et dans lequel le fluide de transfert de chaleur est mis en contact avec un tamis moléculaire.

L'invention concerne également un procédé de production d'électricité au moyen d'un moteur thermique contenant un fluide de transfert de chaleur, ledit procédé comprenant successivement : l'évaporation du fluide de transfert de chaleur, la détente du fluide de transfert de chaleur dans une turbine permettant de générer de l'électricité, la condensation du fluide de transfert de chaleur et la compression du fluide de transfert de chaleur, dans lequel le fluide de transfert de chaleur comprend du trans-1-chloro-3,3,3-trifluoropropène et un composé alcène en C3 à C6, et dans lequel le fluide de transfert de chaleur est mis en contact avec un tamis moléculaire.

Dans certains modes de réalisation, le fluide de transfert de chaleur atteint une température supérieure ou égale à 100°C, de préférence supérieure ou égale à 150°C, de préférence encore supérieure ou égale à 200°C, de préférence encore supérieure ou égale à 220°C.

L'invention concerne également une installation de transfert de chaleur comprenant un circuit de compression de vapeur contenant un fluide de transfert de chaleur, le fluide de transfert de chaleur comprenant du trans-1-chloro-3,3,3-trifluoropropène et un composé alcène en C3 à C6, et le circuit de compression de vapeur étant pourvu d'un tamis moléculaire.

Dans certains modes de réalisation, l'installation est choisie parmi les installations mobiles ou stationnaires de chauffage par pompe à chaleur, de climatisation, de réfrigération, de congélation et les moteurs thermiques.

Dans certains modes de réalisation, le composé alcène est le 2-méthyl-but-2-ène.

Dans certains modes de réalisation, le tamis moléculaire est un adsorbant zéolithique, ou de préférence au moins deux adsorbants zéolithiques disposés en couches.

La présente invention répond au besoin exprimé ci-dessus. Elle fournit plus particulièrement un moyen amélioré permettant de stabiliser le HCFO-1233zdE, notamment dans des systèmes de compression de vapeur tels que des systèmes de climatisation, de réfrigération, de pompe à chaleur et de moteur thermique, tout particulièrement les systèmes comportant un évaporateur noyé, et tout particulièrement dans les applications à température élevée.

Les présents inventeurs ont en effet constaté que les problèmes d'isomérisation et de dégradation du HCFO-1233zeE qui surviennent à haute température sont favorisés par la présence d'air et d'humidité, voire d'acidité, si bien que ces problèmes se produisent dans une certaine mesure malgré la présence d'un stabilisant. L'utilisation d'un tamis moléculaire permet de limiter ou empêcher la présence d'air et d'humidité dans le circuit de réfrigération et, de manière surprenante, n'affecte pas les stabilisants alcènes en C3 à C6.

### DESCRIPTION DE MODES DE REALISATION DE L'INVENTION

L'invention est maintenant décrite plus en détail et de façon non limitative dans la description qui suit.

Sauf mention contraire, dans l'ensemble de la demande les proportions de composés indiquées sont données en pourcentages massiques.

L'invention propose la mise en contact du HCFO-1233zdE avec un composé alcène en C3 à C6 comportant une seule double liaison ainsi qu'avec au moins un tamis moléculaire afin de stabiliser le HCFO-1233zdE, c'est-à-dire de limiter ou d'empêcher son isomérisation en HCFO-1233zdZ, notamment à des températures élevées et notamment lorsque des traces d'humidité et/ou d'air et/ou d'acidité sont présents. Cette mise en contact permet également de stabiliser le HCFO-1233zdE en évitant sa dégradation. Par « *dégradation* », on entend la conversion de molécules de HCFO-1233zd en d'autres espèces. La dégradation du HCFO-1233zdE peut notamment se traduire par une augmentation de la concentration des ions fluorures et chlorures présents dans la composition comprenant le HCFO-1233zdE.

Les composés alcènes selon l'invention sont le propène, les butènes, les pentènes et les hexènes. Les butènes et les pentènes sont préférés. Les pentènes sont encore plus particulièrement préférés.

Les composés alcènes selon l'invention peuvent être à chaîne linéaire ou ramifiée, et de préférence ramifiée.

De préférence, ils présentent une température d'ébullition inférieure ou égale à 100°C, de préférence encore inférieure ou égale à 75 ° C, et de manière plus particulièrement préférée inférieure ou égale à 50°C.

Par « *température d'ébullition* », on entend la température d'ébullition à une pression de 101,325 kPa, telle que déterminée selon la norme NF EN 378-1 d'avril 2008.

De préférence également, ils présentent une température de solidification inférieure ou égale à 0°C, de préférence inférieure ou égale à -25°C, et de manière plus particulièrement préférée inférieure ou égale à -50°C.

La température de solidification est déterminée selon l'*Essai n*° *102: Point de fusion*/*Intervalle de fusion* (Lignes directrices de l'OCDE pour les essais de produits chimiques, Section 1, Éditions OCDE, Paris, 1995, disponible à l'adresse http://dx.doi.org/10.1787/9789264069534-fr).

Des composés alcènes selon l'invention sont notamment :
- le but-1-ène ;
- le cis-but-2-ène ;
- le trans-but-2-ène ;
- le 2-méthyl-prop-1-ène ;
- le pent-1-ène ;
- le cis-pent-2-ène ;
- le trans-pent-2-ène ;
- le 2-méthyl-but-1-ène ;
- le 2-méthyl-but-2-ène ; et
- le 3-méthyl-but-1-ène.

Parmi les composés préférés, on compte notamment le 2-méthyl-but-2-ène, de formule (CH₃)₂C=CH-CH₃ (température d'ébullition de 39°C environ).

Deux ou plus de deux des composés ci-dessus peuvent également être utilisés en combinaison.

Les composés alcènes selon l'invention sont ainsi avantageusement utilisés en association avec du HCFO-1233zd, et plus particulièrement ils sont mélangés avec du HCFO-1233zdE, pour fournir des fluides de transfert de chaleur utilisés dans des applications de transfert de chaleur.

La proportion massique des composés alcènes ci-dessus dans le fluide de transfert de chaleur peut notamment être : de 0,01 à 0,05 % ; ou de 0,05 à 0,1 % ; ou de 0,1 à 0,2 %, ou de 0,2 à 0,3 % ; ou de 0,3 à 0,4 % ; ou de 0,4 à 0,5 % ; ou de 0,5 à 0,6 % ; ou de 0,6 à 0,7 % ; ou de 0,7 à 0,8 % ; ou de 0,8 à 0,9 % ; ou de 0,9 à 1 % ; ou de 1 à 1,2 % ; ou de 1,2 à 1,5 %, ou de 1,5 à 2 % ; ou de 2 à 3 % ; ou de 3 à 4 % ; ou de 4 à 5 %.

Le fluide de transfert de chaleur peut comprendre du HCFO-1233zdE et éventuellement du HCFO-1233zdZ. Avantageusement, la proportion de HCFO-1233zdE, par rapport au total du HCFO-1233zd, est supérieure ou égale à 90 %, ou à 91 %, ou à 92 %, ou à 93 %, ou à 94 %, ou à 95 %, ou à 96 %, ou à 97 %, ou à 98 %, ou à 99 %, ou à 99,1 %, ou à 99,2 %, ou à 99,3 %, ou à 99,4 %, ou à 99,5 %, ou à 99,6 %, ou à 99,7 %, ou à 99,8 %, ou à 99,9 %, ou à 99,91 %, ou à 99,92 %, ou à 99,93 %, ou à 99,94 %, ou à 99,95 %, ou à 99,96 %, ou à 99,97 %, ou à 99,98 %, ou à 99,99 %.

La présence du composé alcène permet de limiter ou d'empêcher une augmentation de la proportion de HCFO-1233zdZ dans la composition au cours du temps et/ou en cas d'application de températures relativement élevées. La présence du composé alcène permet également de limiter la concentration des ions fluorures et chlorures présents dans la composition et plus généralement de limiter la dégradation du HCFO-1233zdE.

De plus, le composé alcène est susceptible d'avoir également une fonction de transfert de chaleur, tout comme le HCFO-1233zdE et les autres composés de transfert de chaleur éventuels. Dans certains modes de réalisation, il est susceptible de former des mélanges azéotropiques ou quasi-azéotropiques avec le HCFO-1233zdE (et le cas échéant avec les autres composés de transfert de chaleur éventuellement présents).

Le fluide de transfert de chaleur peut éventuellement être associé à des additifs divers, pour former avec ceux-ci une composition de transfert de chaleur. Les additifs peuvent notamment être choisis parmi les lubrifiants, les nanoparticules, les stabilisants (différents des composés stabilisants de l'invention), les tensioactifs, les agents traceurs, les agents fluorescents, les agents odorants et les agents de solubilisation.

Le ou les stabilisants, lorsqu'ils sont présents, représentent de préférence au plus 5 % en masse dans la composition de transfert de chaleur. Parmi les stabilisants, on peut citer notamment le nitrométhane, l'acide ascorbique, l'acide téréphtalique, les azoles tels que le tolutriazole ou le benzotriazole, les composés phénoliques tels que le tocophérol, l'hydroquinone, le t-butyl hydroquinone, le 2,6-di-ter-butyl-4-méthylphénol, les époxydes (alkyl éventuellement fluoré ou perfluoré ou alkényl ou aromatique) tels que les n-butyl glycidyl éther, hexanediol diglycidyl éther, allyl glycidyl éther, butylphénylglycidyl éther, les phosphites, les phosphonates, les thiols, le limonène, le limonène oxyde et les lactones.

A titre de lubrifiants on peut notamment utiliser des huiles d'origine minérale, des huiles de silicone, des paraffines d'origine naturelle, des naphtènes, des paraffines synthétiques, des alkylbenzènes, des poly-alpha oléfines, des polyalkène glycols, des polyol esters et/ou des polyvinyléthers.

Dans certains modes de réalisation avantageux de l'invention, la composition de l'invention est toutefois dépourvue de lubrifiant.

A titre de nanoparticules on peut notamment utiliser les nanoparticules de charbon, les oxydes métalliques (cuivre, aluminium), TiO₂, Al₂O₃, MoS₂...

A titre d'agents traceurs (susceptibles d'être détectés) on peut citer les hydrofluorocarbures deutérés ou non, les hydrocarbures deutérés, les perfluorocarbures, les fluoroéthers, les composés bromés, les composés iodés, les alcools, les aldéhydes, les cétones, le protoxyde d'azote et les combinaisons de ceux-ci. L'agent traceur est différent du ou des composés de transfert de chaleur composant le fluide de transfert de chaleur.

A titre d'agents de solubilisation, on peut citer les hydrocarbures, le diméthyléther, les polyoxyalkylène éthers, les amides, les cétones, les nitriles, les chlorocarbures, les esters, les lactones, les aryl éthers, les fluoroéthers et les 1,1,1-trifluoroalcanes. L'agent de solubilisation est différent du ou des composés de transfert de chaleur composant le fluide de transfert de chaleur.

A titre d'agents fluorescents, on peut citer les naphthalimides, les perylènes, les coumarines, les anthracènes, les phénanthracènes, les xanthènes, les thioxanthènes, les naphthoxanhtènes, les fluorescéines et les dérivés et combinaisons de ceux-ci.

A titre d'agents odorants, on peut citer les alkylacrylates, les allylacrylates, les acides acryliques, les acrylesters, les alkyléthers, les alkylesters, les alcynes, les aldéhydes, les thiols, les thioéthers, les disulfures, les allylisothiocyanates, les acides alcanoïques, les amines, les norbornènes, les dérivés de norbornènes, le cyclohexène, les composés aromatiques hétérocycliques, l'ascaridole, l'o-méthoxy(méthyl)-phénol et les combinaisons de ceux-ci.

Le fluide de transfert de chaleur (ainsi que la composition de transfert de chaleur qui le contient) peut également comprendre au moins un autre composé de transfert de chaleur, en plus du HCFO-1233zd. Un tel autre composé de transfert de chaleur optionnel peut être notamment un composé hydrocarbure, éther, hydrofluoroéther, hydrofluorocarbure, hydrochlorofluorocarbure, hydrofluorooléfine, hydrochlorooléfine ou hydrochlorofluorooléfine.

A titre d'exemple, ledit autre composé de transfert de chaleur peut être choisi parmi le 1,1,1,4,4,4-hexafluorobut-2-ène (HFO-1336mzz, isomère E ou Z), le 3,3,4,4,4-pentafluorobut-1-ène (HFO-1345fz), le 2,4,4,4-tétrafluorobut-1-ène (HFO-1354mfy), le 1,1,1,3,3-pentafluoropropane (HFC-245fa), le 2,3,3,3-tétrafluoropropène (HFO-1234yf), le 1,3,3,3-tétrafluoropropène (HFO-1234ze), le difluorométhane (HFC-32), le 1,1,1,2-tétrafluoroéthane (HFC-134a), le 1,1,2,2-tétrafluoroéthane (HFC-134), le 1,1-difluoroéthane (HFC-152a), le pentafluoroéthane (HFC-125), le 1,1,1,3,3-pentafluorobutane (HFC-365mfc), le méthoxynonafluorobutane (HFE7100), le butane (HC-600), le 2-méthylbutane (HC-601a), le pentane (HC-601), l'éthyl éther, le méthyl acétate et les combinaisons de ceux-ci.

Dans le fluide de transfert de chaleur, le HCFO-1233zd peut représenter notamment, en masse, de 50 à 55 % du fluide ; ou de 55 à 60 % du fluide ; ou de 60 à 65 % du fluide ; ou de 65 à 70 % du fluide ; ou de 70 à 75 % du fluide ; ou de 75 à 80 % du fluide ; ou de 80 à 85 % du fluide ; ou de 85 à 90 % du fluide ; ou de 90 à 95 % du fluide ; ou de 95 à 99 % du fluide ; ou de 99 à 99,5 % du fluide ; ou de 99,5 à 99,9 % du fluide ; ou plus de 99,9 % du fluide. La teneur en HFO-1233zd peut également varier dans plusieurs des intervalles ci-dessus : par exemple de 50 à 55 % et de 55 à 60 %, c'est-à-dire de 50 à 60 %, etc.

La composition de transfert de chaleur/le fluide de transfert de chaleur décrit ci-dessus est mis en contact, selon l'invention, avec au moins un tamis moléculaire qui est de préférence placé dans une cartouche.

Le tamis moléculaire est de préférence un adsorbant zéolithique.

Les adsorbants zéolithiques, ou plus simplement les zéolithes, utilisables dans le cadre de la présente invention, peuvent être de tout type connu de l'homme du métier et notamment les zéolithes de type A, les zéolithes de type faujasite, c'est-à-dire les zéolithes X et LSX (pour « *Low silica X* »), et les zéolithes de type Y. Il est entendu que ces différentes zéolithes peuvent être utilisées seules ou en mélange de deux ou plusieurs d'entre elles.

Les zéolithes sont typiquement des composés à base d'alumino-silicates, cristallins et poreux, qui possèdent une structure cristalline tridimensionnelle constituée par un assemblage de tétraèdres de SiO₄ et AlO₄ reliés entre eux grâce à la mise en commun d'un ou plusieurs atomes d'oxygène. Ces composés forment ainsi des réseaux cristallins contenant des pores de taille nanométrique.

Ces édifices contiennent généralement des cations pour rendre le système électriquement neutre, ces cations étant le plus souvent des cations comprenant du sodium, du potassium ou du calcium, mais aussi du baryum, des terres rares ou encore des mélanges de deux ou plusieurs de ces cations en toutes proportions.

En général, les zéolithes utilisées sont des zéolithes synthétiques obtenues sous forme de poudre à l'issue d'un processus de nucléation et cristallisation de gels d'alumino-silicates. Les zéolithes naturelles, telles que par exemple les zéolithes de type clinoptilolite, mordenite ou chabazite, dont les utilisations principales sont généralement les opérations de purification ou de déshydratation, peuvent être également utilisées.

Selon un mode de réalisation préféré de la présente invention, la ou les zéolithe(s) utilisée(s) comprennent les zéolithes de type A, les zéolithes de type faujasite, c'est-à-dire les zéolithes X, LSX, et les zéolithes de type Y.

Les zéolithes répondent à la formule générale suivante :

M_{x/n}[(AlO₂)ₓ(SiO₂)_{y}] . w H₂O

dans laquelle :
M représente un ou plusieurs cations de valence totale n, w représente le nombre de molécules d'eau, le ratio (y/x) est compris entre 1 et 5 selon les structures des zéolithes et la somme (x+y) représente le nombre de tétraèdres par cellule unitaire.

La structure et les propriétés de la zéolithe A sont connues et abondamment décrites dans la littérature, notamment dans l'ouvrage de Donald W. Breck, "Zeolite Molecular Sieves", éditions John Wiley and Sons, (1974), pp. 83 et suivantes, et par les documents US 2,882,243 et FR 1 257 034.

Le rapport Si/AI dans les zéolithes A est toujours proche de 1. La présence de cations sodium permet d'assurer l'électro-neutralité de la structure.

La modification de la nature des cations par échange de tout ou partie peut s'accompagner d'une variation de la taille des pores ou d'une modification de la sélectivité par création d'interactions spécifiques avec les molécules adsorbées et ainsi changer les propriétés d'adsorption.

Ainsi pour la zéolithe A qui, sous forme sodique après la synthèse, présente une ouverture de pores de 4A, il est possible de réaliser divers échanges cationiques A, afin de lui conférer les propriétés souhaitées.

Fréquemment, il s'agit de cations alcalins ou alcalino-terreux tels que le lithium (Li⁺), le potassium (K⁺), le césium (Cs⁺), le magnésium (Mg²⁺), le calcium (Ca²⁺), le strontium (Sr²⁺), le baryum (Ba²⁺), le cérium (Ce³⁺), ou d'autres tels que les terres rares ou les métaux, par exemple le lanthane (La²⁺/La³⁺), l'argent (Ag⁺), le cuivre (Cu²⁺), le nickel (Ni²⁺), le zinc, le fer, le chrome, et autres.

Ainsi, selon le type d'échange cationique réalisé, la zéolithe A peut par exemple être transformée soit :
- en la forme calcique par échange avec un sel de calcium en solution aqueuse, afin d'obtenir une zéolithe dont les pores ont une ouverture effective de 5A ;
- en la forme potassium par échange avec un sel de potassium en solution aqueuse afin d'obtenir une zéolithe dont les pores ont une ouverture effective de 3A ;
- en différentes formes en mélangeant des solutions aqueuses de sels de lithium, calcium ou de potassium par exemple.

Par zéolithe 4A, on entend ici une zéolithe de type A dont essentiellement tous les sites cationiques échangeables sont occupés par des cations sodium Na⁺ (forme sodique après synthèse).

Par zéolithe 5A, on entend ici une zéolithe de type A dont au moins 40 % des sites cationiques (rapportés en équivalents) sont occupés par des cations Ca²⁺, le reste des sites pouvant être occupés par des cations sodium Na⁺, mais on ne sortirait pas du cadre de l'invention si d'autres cations étaient présents comme décrit précédemment.

Par zéolithe 3A, on entend ici une zéolithe de type A dont 20 à 70 % (rapportés en équivalents) des sites cationiques échangeables sont occupés par des cations potassium, mais on ne sortirait pas de l'invention si d'autres cations étaient présents comme décrit précédemment.

Les faujasites constituent un groupe d'espèces minérales caractérisées par leur structure topographique cristallographique, qui sont notamment décrites dans l'ouvrage de Donald W. Breck "Zeolite Molecular Sieves", éditions John Wiley and Sons, (1974), pp. 92 et suivantes.

La règle dite de Lowenstein leur impose un rapport molaire Si/Al supérieur ou tout au moins égal à 1. On a coutume de distinguer :
- les faujasites X classiques de rapport Si/Al > 1,15 ;
- les faujasites LSX (acronyme anglais de « *Low Silica X* ») ou faujasites à faible teneur en silice qui sont des espèces zéolithiques de type X de rapport atomique Si/Al inférieur ou égal à 1,15, de préférence égal à 1 ± 0,05 (les valeurs inférieures à l'unité traduisent les incertitudes analytiques sur la mesure de ce rapport et les valeurs supérieures, soit la même incertitude analytique, soit un écart tolérable de pureté du produit) ; et
- les faujasites Y avec un rapport Si/Al > 1,5.

La cellule élémentaire de la zéolithe X est un tétraèdre dont les sommets sont occupés par des polyèdres de même type que ceux présents dans la zéolithe A, chacun étant connecté à quatre autres polyèdres grâce à une sous-structure octaédrique, formée par un double-cycle contenant huit atomes d'oxygène. Le centre de chaque arête est toujours occupé par un atome d'oxygène, tandis que les atomes de silicium et d'aluminium occupent les différents sommets des polyèdres. Une forme préférée de la zéolithe X est la zéolithe 13X qui a la formule chimique suivante :

Na86 [(AlO₂)₈₆ (SiO₂)₁₀₆] . H₂O

Les zéolithes X et Y sont sous forme sodique après leurs synthèses : NaX, NaY, la zéolithe LSX après synthèse est sous forme NaKLSX.

Ces zéolithes peuvent également subir des traitements d'échange ou de modification et l'on cherche en général à remplacer les cations alcalins (Na, K) par exemple par des protons, des ions alcalins, des ions alcalinoterreux, des ions de terres rares ou de métaux tels que par exemple cités précédemment.

Les zéolithes de l'invention peuvent se présenter sous forme de poudre ou d'agglomérés. Par agglomération, on entend la mise en forme de la poudre de zéolithe à l'aide d'un liant minéral et/ou organique. Cette mise en forme d'agglomérés peut être réalisée selon toute méthode connue de l'homme du métier. Par exemple, les agglomérés peuvent être sous forme de plaquettes, de billes de quelques nanomètres à quelques millimètres de diamètre moyen, de fils ou d'extrudés, de barres, de joncs, ou encore de pièces moulées de tailles et de formes diverses, que l'on peut génériquement nommer « *cores* » selon la terminologie anglaise, et autres.

Cette mise en forme se fait par mélange d'un mélange pâteux de zéolithe(s) de liant(s) et éventuellement d'un ou plusieurs additifs destinés par exemple à faciliter la manipulation de la pâte par modification de la rhéologie et/ou du pouvoir collant. Ce liant, le plus souvent inerte, est destiné à assurer la cohésion des cristaux de zéolithe(s) entre eux.

Parmi les liants minéraux on peut utiliser de l'alumine, la montmorillonite (bentonite), l'attapulgite, la sépiolite, des argiles zéolithisables, telles que celles choisies parmi les kaolins, les kaolinites, les nacrites, les dickites, les halloysites, les métakaolins, les argiles colloïdales, par exemple de type Attagel ou encore d'autre minéraux ou zéolithes naturelles (clinoptilolite, mordenite ou chabazite), des terres de diatomées, du talc, et autres liants minéraux connus de l'homme du métier, qui peuvent être utilisés seuls ou en mélange de deux ou plusieurs d'entre eux.

Parmi les liants organiques qui peuvent être utilisés seuls ou en association avec les liants minéraux cités précédemment, on entend toute matrice polymère connue en soi de l'homme du métier spécialiste des polymères. Elle peut comprendre un homopolymère et/ou copolymère thermoplastique et/ou thermodurcissable, par exemple, et à titre non limitatif, polyuréthane, polymères fluorés, tels que PVDF, résines époxydes, et autres. Ces polymères peuvent se présenter sous toutes formes, et par exemple sous forme de mousse, expansée ou semi-expansée.

A titre d'exemples de matrices polymères on peut citer celles décrites dans la demande internationale WO 2010/063975, dans laquelle la matrice polymère comprend une polyoléfine (par exemple de type polyéthylène, polypropylène, et autres), des élastomères (tels que ceux de type copolymères acrylates, par exemple copolymère éthylène/acrylate de butyle), un polyamide, un polyester ou encore un mélange de deux ou plus de ces polymères.

La matrice polymère peut également comprendre, en totalité ou en partie, un ou plusieurs polymères, homo- et/ou copolymères, susceptibles de former un assemblage supramoléculaire. Par assemblage supramoléculaire, on entend des polymères, homo- et/ou copolymères, susceptibles de s'associer entre eux au moyen de liaisons hydrogènes.

Parmi les polymères dits « *supramoléculaires* », on peut citer, à titre d'exemples non limitatifs, les polymères semi-cristallins, et notamment ceux formés par assemblage supramoléculaire de composés résultants de la condensation d'un acide gras et/ou d'un dimère d'acide gras et/ou d'un trimère d'acide gras et d'au moins une amine associative (susceptible de former des liaisons hydrogènes) choisie parmi la 1-(2-aminoéthyl)-imidazolidin-2-one (UDETA), la 1-(2-[(2-aminoéthyl)amino]éthyl)imidazolidone (UTETA), la 1-(2-{2-[(2-aminoéthylamino]éthyl}amino)éthyl]imidazolidone (UTEPA), et la N-(6-aminohexyl)-N'-(6-méthyl-4-oxo-1,4-dihydropyrimidin-2-yl)urée (UPy), et leurs mélanges.

Outre les liants minéraux et/ou organiques, on peut ajouter aux zéolithes un ou plusieurs additifs communément employés et connus de l'homme du métier, et par exemple les additifs choisis parmi la silice, la silice colloïdale, la cellulose, l'amidon de maïs ou tout autre type d'agent porogène.

D'une manière générale, les zéolithes mises en œuvre dans la présente invention peuvent se présenter sous toutes formes, et par exemple sous forme d'aggloméré zéolithique à liant organique comme décrit dans la demande internationale WO 2010/063975 pour l'élimination d'eau dans l'application double vitrage, ou encore comme décrit dans les brevets US 2,583,812, US 4,013,566, et les demandes de brevets US 2001/0014707 et EP 1 566 600 dans lesquels sont divulgués des solides à base de zéolithes et de polymères destinés au séchage des fluides réfrigérants.

Au sens de l'invention, l'aggloméré zéolithique à base de liant organique est généralement obtenu à partir d'un compound (mélange, puis mis en forme par exemple par extrusion, moulage, extrusion-moulage, injection-extrusion ou toute autre technique connue de l'homme du métier permettant l'obtention d'un article sous forme solide à partir d'au moins une matrice polymère fondue.

Dans un mode de réalisation, le matériau adsorbant selon la présente invention peut comprendre en outre un ou plusieurs additifs, couramment utilisés dans les techniques de compoundage. Des exemples non limitatifs de tels additifs peuvent être choisis parmi les stabilisants UV, les pigments, les colorants, les antioxydants, les modifiants chocs, les matériaux à changement de phase (MCP), les agents ignifugeants, les agents odorants, la cellulose, et autres, seuls ou en mélange.

Les composés zéolithiques, qu'ils soient sous forme agglomérée ou sous forme de poudre (i.e. forme non agglomérée), utilisables dans le cadre de la présente invention peuvent éventuellement être soumis à un traitement par imprégnation, par exemple imprégnation en phase aqueuse au moyen d'hydroxyde(s) de métal(aux) alcalin(s)et/ou alcalino-terreux ou par incorporation de ce(s) d'hydroxyde(s) et/ou carbonate(s) et/ou de sel(s) de métal(aux) alcalin(s) et/ou alcalino-terreux avant, après ou lors de l'étape d'agglomération et/ou avant, après ou lors de l'étape de mise en forme.

Cette opération d'imprégnation vise à imprégner les zéolithes ou les agglomérés zéolithiques par un ou plusieurs métaux, non-métaux et/ou terres rares, par exemple choisis parmi l'aluminium, le scandium, le gallium, le fer (III), le chrome (III), l'indium, l'yttrium, les lanthanides ou plus généralement les terres rares, seules ou en mélange et/ou d'ions divalents choisis parmi les ions calcium, strontium, zinc, cuivre, chrome (II), fer (II), manganèse, nickel, cobalt, seuls ou en mélange.

Selon un autre aspect, il doit être compris que les traitements visant à opérer les échanges ou modifications cationiques définis plus haut peuvent être effectués soit sur les cristaux de zéolithes (poudre) soit sur les zéolithes déjà mises en forme (agglomérées, imprégnées, et autres), soit encore avant et après mise(s) en forme des adsorbants zéolithiques.

Selon un mode de réalisation préféré de la présente invention, les adsorbants zéolithiques sont à base de zéolithe A ou de zéolithe faujasite, et de manière encore plus préférée, les adsorbants zéolithiques sont à base de zéolithe(s) A (3A, 4A ou 5A) et/ou de zéolithe 13X et de manière encore préférée soit de la poudre de zéolithe 3A soit des agglomérés à base de poudre de zéolithe A, échangée au potassium, l'échange potassium pouvant être effectué soit sur la poudre de départ et/ou sur l'aggloméré final.

Selon certains modes de réalisation, deux ou plus de deux adsorbants zéolithiques peuvent être utilisées. Ces modes de réalisation sont préférés étant donné les propriétés différentes des zéolithes vis-à-vis de l'adsorption de l'air et de l'eau. Plus précisément, certaines zéolithes telles que la zéolithe 3A sont particulièrement efficaces pour adsorber de l'eau tandis que d'autres zéolithes telles que la zéolithe 5A ou la zéolithe 13X sont particulièrement efficaces pour adsorber de l'air. Ainsi, leur utilisation en combinaison peut offrir une protection plus efficace contre l'air et l'eau. Par exemple, les deux ou plus de deux adsorbants zéolithiques peuvent être de la zéolithe 13X et de la zéolithe 3A, ou de la zéolithe 3A et de la zéolithe 5A, ou de la zéolithe 13X, de la zéolithe 3A et de la zéolithe 5A.

Selon certains modes de réalisation préférés, les deux ou plus de deux adsorbants zéolithiques peuvent être placés sous forme de couches dans une cartouche. Par exemple, la zéolithe 3A peut constituer une première couche suivie par une deuxième couche comprenant de la zéolithe 5A et/ou de la zéolithe 13X. Par « *première couche* » on entend la couche qui est la première à être en contact avec le flux du fluide de transfert de chaleur dans la direction du fluide.

Selon un autre mode de réalisation préféré, les adsorbants zéolithiques utilisables dans le cadre de la présente invention sont à base de zéolithes A échangées au potassium, et dont le taux d'échange est compris entre 20 % et 70 % (rapportés en équivalents molaires) de la totalité des sites cationiques échangeables, de manière préférée compris entre 30 % et 70 %, de manière encore préférée entre 40 % et 70 % et de manière tout particulièrement préférée entre 50 % et 70 %.

Lorsque les adsorbants zéolithiques sont des agglomérés de zéolithes, le liant d'agglomération est de manière préférée de l'attapulgite, de l'attapulgite colloïdale, de la sépiolite, de la bentonite, du kaolin, de l'halloysite, ces liants d'agglomération pouvant être utilisés seuls ou en mélange(s) avec d'autres argiles ou zéolithes naturelles (clinoptilolite, mordenite ou chabazite). De manière préférée le liant d'agglomération contient majoritairement de l'attapulgite ou du kaolin et de manière encore préférée de l'attapulgite.

A titre d'exemples non limitatifs d'adsorbants zéolithiques qui peuvent être utilisées dans le cadre de la présente invention, on peut citer les adsorbants commercialisés par CECA sous les noms Siliporite^{®} H3Ri, Siliporite^{®} NK10, Siliporite^{®} NK30, Siliporite^{®} SA 1720, Siliporite^{®} NK20, Siliporite^{®} G5 XP, ceux commercialisés par ZEOCHEM sous les dénominations Purmol^{®} 3ST (3A), Purmol^{®} 4ST (A), Zeochem^{®} Z4-01, Zeochem^{®} 4A-8BL, ou encore ceux commercialisés par GRACE sous les noms Sylosiv^{®}, Cryosiv^{®}, ou par UOP sous les dénominations Molsiv^{™} 3A, Molsiv^{™} 4A, Molsiv^{™} 5A, XH-7^{™}, XH-9^{™} et XH-11^{™}.

Selon certains modes de réalisation, un ou plusieurs composés capables d'éliminer les traces d'acidité présentes dans le fluide de transfert de chaleur peuvent également être utilisés en combinaison avec les tamis moléculaires, et notamment avec les adsorbants zéolithiques. Ce ou ces composés peuvent être choisis parmi les oxydes de métaux tels que l'oxyde l'aluminium, les oxydes de métaux alcalino-terreux, les oxydes de métaux alcalins, les hydroxydes de métaux tels que l'hydroxyde d'aluminium, les hydroxydes de métaux alcalino-terreux, les hydroxydes de métaux alcalins, les minéraux d'aluminosilicates tels que l'andalousite, le disthène, la sillimanite, l'aluminosilicate de calcium, l'aluminosilicate de sodium et l'oxyde de silice. De préférence, ce composé est l'oxyde d'aluminium et encore de préférence ce composé est l'oxyde d'aluminium activé. L'oxyde d'aluminium activé est une substance poreuse et granulaire qui peut être fabriquée à partir de l'hydroxyde d'aluminium en le déshydroxylant de manière à produire un matériau hautement poreux. L'alumine activée peut avoir une surface supérieure à 200 m²/g.

Les tamis moléculaires tels que les adsorbants zéolithiques adsorbent l'air et l'eau ce qui permet la diminution de la réactivité du milieu et en conséquence la diminution de l'acidité. Cependant, il est préféré, afin d'éliminer toute trace d'acidité, d'utiliser également un ou plusieurs composés mentionnés ci-dessus.

Dans le cas ou un ou plusieurs composés capables d'éliminer les traces d'acidité sont utilisés, ce ou ces composés peuvent également être placés sous forme d'au moins une couche dans la cartouche comprenant le ou les adsorbants zéolithiques. La couche comprenant le ou les composés capables d'éliminer les traces d'acidité est de préférence placée en amont de la ou les couches comprenant les tamis moléculaires (notamment adsorbants zéolithiques) et en particulier en amont de la première couche comprenant l'adsorbant zéolithique (par rapport à la direction de circulation du flux de fluide de transfert de chaleur).

Alternativement, ce ou ces composés peuvent être utilisés en mélange avec le ou les tamis moléculaires.

L'invention est de préférence mise en œuvre dans une installation de transfert de chaleur comprenant un système de compression de vapeur.

L'installation de transfert de chaleur est utilisée pour un procédé de transfert de chaleur. Le procédé de transfert de chaleur peut être un procédé de chauffage ou de refroidissement d'un fluide ou d'un corps.

L'installation de transfert de chaleur peut également être utilisée dans un procédé de production de travail mécanique ou d'électricité, notamment conformément à un cycle de Rankine.

Pour les applications de chauffage et de refroidissement, le système de compression de vapeur comprend au moins un évaporateur, un compresseur, un condenseur et un détendeur, ainsi que des lignes de transport de fluide de transfert de chaleur entre ces éléments. L'évaporateur et le condenseur comprennent un échangeur de chaleur permettant un échange de chaleur entre le fluide de transfert de chaleur et un autre fluide ou corps.

A titre de compresseur, on peut utiliser notamment un compresseur centrifuge à un ou plusieurs étages ou un mini-compresseur centrifuge. Les compresseurs rotatifs, spirales, à piston ou à vis peuvent aussi être utilisés. Le compresseur peut être entraîné par un moteur électrique ou par une turbine à gaz (par exemple alimentée par les gaz d'échappement d'un véhicule, pour les applications mobiles) ou par engrenage.

Le système de compression de vapeur fonctionne alors selon un cycle classique de compression de vapeur. Le cycle comprend le changement d'état du fluide de transfert de chaleur d'une phase liquide (ou diphasique liquide/vapeur) vers une phase vapeur à une pression relativement faible, puis la compression du fluide en phase vapeur jusqu'à une pression relativement élevée, le changement d'état (condensation) du fluide de transfert de chaleur de la phase vapeur vers la phase liquide à une pression relativement élevée, et la réduction de la pression pour recommencer le cycle.

De préférence, le tamis moléculaire est disposé dans une cartouche comme décrit ci-dessus qui est placée dans le système de compression de vapeur ou dans un système de type cycle organique de Rankine. Le fluide traversant la cartouche peut être à l'état vapeur, à l'état liquide ou à l'état diphasique (liquide et vapeur). La cartouche peut être placée en amont ou en avale d'un détendeur, d'un compresseur, d'une turbine ou entre une série d'échangeurs. De préférence, la cartouche peut être placée dans la zone à plus faible température soit entre la turbine et la pompe liquide dans un cycle organique de Rankine. De préférence, le fluide traversant la cartouche est à l'état liquide.

L'installation de transfert de chaleur peut également éventuellement comprendre au moins un circuit de fluide caloporteur utilisé pour transmettre la chaleur (avec ou sans changement d'état) entre le circuit de fluide de transfert de chaleur et le fluide ou corps à chauffer ou refroidir.

L'installation de transfert de chaleur peut également éventuellement comprendre deux systèmes de compression de vapeur (ou plus), contenant des fluides de transfert de chaleur identiques ou distincts. Par exemple, les systèmes de compression de vapeur peuvent être couplés entre eux.

Les procédés et installations de refroidissement selon l'invention comprennent les procédés et installations de climatisation (avec des installations mobiles, par exemple dans des véhicules, ou stationnaires), de réfrigération (avec des installations mobiles par exemple dans les containers, ou stationnaires) et de congélation ou de cryogénie.

Les installations de chauffage selon l'invention comprennent les pompes à chaleur.

Pour les applications de production de travail mécanique ou d'électricité, l'installation de transfert de chaleur est un moteur thermique, qui comprend au moins un évaporateur, un organe de détente, un condenseur et une pompe, ainsi que des lignes de transport de fluide de transfert de chaleur entre ces éléments. L'installation de transfert de chaleur peut alors fonctionner selon un cycle de Rankine.

De préférence, le tamis moléculaire est disposé dans une cartouche comme décrit ci-dessus placée dans le système thermodynamique, de préférence après l'organe de détente. Ainsi, le fluide de transfert de chaleur en sortant de l'organe de détente traverse la cartouche comprenant le tamis moléculaire.

A titre d'organe de détente, on peut notamment utiliser des turbines à un ou plusieurs étages ou bien des détendeurs. A titre d'exemple, des détendeurs rotatifs, spirales, à piston ou à vis peuvent aussi être utilisés.

Il est possible d'utiliser tout type d'échangeur de chaleur pour la mise en œuvre des fluides de transfert de chaleur selon l'invention, et notamment des échangeurs de chaleur à co-courant ou, de préférence, des échangeurs de chaleur à contre-courant.

En particulier, l'évaporateur utilisé dans le cadre de l'invention peut être un évaporateur à surchauffe ou un évaporateur noyé. Dans un évaporateur à surchauffe, la totalité du fluide de transfert de chaleur est évaporé à la sortie de l'évaporateur, et la phase vapeur est surchauffée.

Dans un évaporateur noyé, le fluide de transfert de chaleur sous forme liquide ne s'évapore pas complètement. Un évaporateur noyé comporte un séparateur de phase liquide et de phase vapeur.

L'invention est particulièrement utile lorsqu'un tel évaporateur est utilisé. En effet, les stabilisants de l'état de la technique à température d'ébullition élevée sont inefficaces lorsqu'un tel évaporateur est employé, car ils se concentrent dans l'évaporateur et ne migrent pas avec le fluide de transfert de chaleur au condenseur.

Le composé stabilisant alcène n'est pas ou pratiquement pas piégé par le tamis moléculaire. Ainsi, l'action conjointe du composé alcène et du tamis moléculaire permet l'utilisation du fluide de transfert de chaleur à des températures élevées en limitant l'isomérisation et la dégradation du fluide.

L'invention est donc également particulièrement utile lorsqu'une température élevée existe en au moins un point du circuit de fluide, et plus particulièrement une température supérieure ou égale à 100°C, ou à 110°C, ou à 120°C, ou à 130°C, ou à 140°C, ou à 150°C, oà 160°C, ou à 170°C, ou à 180°C, ou à 190°C, ou à 200°C, ou à 210°C, ou à 220°C, ou à 230°C, ou à 240°C, ou à 250°C, ou à 260°C, ou à 270°C. En effetce sont dans ces conditions que le HCFO-1233zdE est le plus susceptible de se convertir en HCFO-1233zdZ ou de se dégrader.

En particulier, dans les appareils de climatisation, la température générale de fonctionnement est inférieure à 100°C ;mais des points chauds à la sortie du compresseur peuvent atteindre des températures supérieures à 100°C, affectant le fluide de transfert de chaleursur une faible proportion de sa durée de circulation complète (par exemple moins de 1 %).

Dans les pompes à chaleur, la température de condensation peut atteindre 160°C environ. Dans ce cas, le fluide de transfert de chaleur peut être à une température de 160°C environ sur une proportion importante de sa durée de circulation complète (par exemple environ 50 %). De plus, des points chauds entre 150 et 200°C peuvent également être constatés à la sortie du compresseur. L'impact d'une durée de séjour longue à des températures supérieures à 100°C et l'existence de points à des températures pouvant avoisiner voire dépasser les 200°C nécessitent doncun stabilisant.

Dans les cycles moteurs pour la production d'électricité de type cycle organique de Rankine, la température peut atteindre 165°C. Dans ce cas, le fluide de transfert de chaleur peut être à une température de 165°C environ ou plus sur une proportion importante de sa durée de circulation complète (par exemple environ 50 %). De plus, des points chauds entre 180 et 250°C, ou plus de 250°C, peuvent également être constatés à lentrée de la turbine. L'impact d'une durée de séjour longue à des températures supérieures à 100°C et l'existence de points à des températures pouvant avoisiner voire dépasser les 250°C nécessitent donc un stabilisant.

De préférence également, dans l'installation de transfert de chaleur, la température de la composition utilisée en tant que fluide de transfert de chaleur reste supérieure à la température de solidification du composé alcène, afin d'éviter tout dépôt de matière solide dans le circuit.

Grâce à la capacité de travailler à des températures plus élevées, l'invention permet également l'augmentation de la surchauffe du fluide de transfert de chaleur. Cette augmentation de surchauffe a comme résultat une augmentation du rendement et ainsi une amélioration des performances du système.

Ainsi, la surchauffe peut valoir de 1 à 90°C, et de préférence de 10 à 80°C. Par exemple, la surchauffe peut être augmentée de 1 à 5°C ; ou de 5 à 10°C ; ou de 10 à 15°C ; ou de 15 à 20°C ; ou de 20à 25°C ; ou de 25 à 30°C ; ou de 30 à 35°C ; ou de 35 à 40°C ; ou de 40 à 45 pu de 45 à 50°C ; ou de 50 à 55°C; ou de 55 à 60°C; ou de 60 à 65°C; oude 65 à 70°C; ou de 70 à 75°C; ou de 75 à 80°C; ou de 80 à 85°C; ou de 85à 90°C.

On désigne par « *surchauffe* » (équivalent ici à « *surchauffe à l'évaporateur* ») le différentiel de température entre la température maximale atteinte par le fluide de transfert de chaleur avant le compresseur ou la turbine (c'est-à-dire la température maximale atteinte par le fluide de transfert de chaleur à l'issue de l'étape de surchauffe qui succède à l'évaporation) et la température de fin d'évaporation.

### EXEMPLES

Les exemples suivants illustrent l'invention sans la limiter.

### Exemple 1-Etude de stabilité thermique du HCFO-1233zdE

On considère un cycle organique de Rankine travaillant entre une température ambiante de 30°C et une température de source chaude entre 300 et 600°C et utilisant du HCFO-1233zdE comme fluide de transfert de chaleur. La température du HCFO-1233zdE peut atteindre une température supérieure à 200°C à la sortie de l'évaporateur.

Le 2-méthyl-but-2-ène est utilisé en tant que composé alcène.

Les résultats de stabilité thermique à 220°C dans ces tubes scellés sont présentés dans le tableau ci-dessous :

| Produit | Acidité (F- et Cl⁻) ppm |
|---|---|
| HCFO-1233zdE + 0,5 %m 2-méthyl-but-2-ène + filtration de l'eau et de l'air sur du tamis | < 150 |
| HCFO-1233zdE + 0,5 %m 2-méthyl-but-2-ène | 800 à 1200 |
| + 0,3 %m d'air | |
| HCFO-1233zdE + 0,5 %m 2-méthyl-but-2-ène + 400 ppm d'eau | 300 à 450 |

Ainsi, les résultats montrent que le 2-méthyl-but-2-ène est capable de réduire le phénomène d'acidité grâce à l'action des tamis sur l'eau et l'air. Sans utilisation de tamis, les résultats montrent que la présence d'eau et d'air impacte la stabilité de cette composition à très haute température (> 200°C) malgré la présence du 2-méthyl-but-2-ène.

### Exemple 2-Utilisation d'une cartouche comprenant un adsorbant zéolithique

Une cartouche comprenant un tamis moléculaire 3A comme adsorbant est placée dans des conditions représentatives de la sortie de la turbine d'un cycle organique de Rankine.

Un mélange de fluide de transfert de chaleur (HCFO-1233zdE) ayant une masse totale de 1400 g a été préparé, en y ajoutant 0,5 % massique de 2-méthyl-but-2-ène et 250 ppm d'eau.

La cartouche comprend 200 g de tamis moléculaire 3A.

Le fluide de transfert de chaleur se trouvant en phase liquide traverse la cartouche à une température de 80°C et une pression de 7 bar.

Le fluide de transfert de chaleur a été récupéré et analysé après son passage à travers la cartouche de tamis moléculaire.

Il a été trouvé que la teneur en eau du fluide de transfert de chaleur a été diminuée à 15 ppm et que la totalité du composé alcène 2-méthyl-but-2-ène a été récupéré dans le fluide de transfert de chaleur.

### Exemple 3-Performances du cycle organique de Rankine

Une analyse des performances d'un cycle organique de Rankine fonctionnant avec du HCFO-1233zdE, avec une température d'évaporation de 150°C et une température de condensation de 40°C aété effectuée dans une installation selon l'invention.

Les résultats sont présentés dans le tableau ci-dessous :

| Surchauffe (°C) | Tₘₐₓ évaporateur (°C) | Tₘₐₓ source de chaleur (°C) | Capacité volumétrique (%) | Rendement (%) |
|---|---|---|---|---|
| 0 | 160 | 165 | 100,00 | 100,00 |
| 3 | 163 | 168 | 96,73 | 102,56 |
| 13 | 173 | 178 | 90,61 | 108,92 |
| 23 | 183 | 188 | 87,22 | 113,86 |
| 33 | 193 | 198 | 84,94 | 118,11 |
| 43 | 203 | 208 | 83,26 | 121,93 |
| 53 | 213 | 218 | 81,97 | 125,45 |
| 65 | 225 | 230 | 80,75 | 129,38 |
| 66 | 226 | 231 | 80,66 | 129,70 |
| 67 | 227 | 232 | 80,57 | 130,01 |
| 68 | 228 | 232 | 80,48 | 130,32 |

Les résultats de la capacité volumétrique et du rendement sont donnés en pourcentage par rapport aux résultats obtenus à 0°C de surchauffe.

Les résultats ci-dessus montrent que malgré une diminution de la capacité volumétrique, l'augmentation de la surchauffe du fluide de transfert de chaleur de 0 à 68°C (qui est permise grâce à l'invention) permet une augmentation du rendement de 30 %.

## Revendications

1. Utilisation conjointe d'un composé alcène en C3 à C6 et comportant une seule double liaison et d'au moins un tamis moléculaire pour limiter ou empêcher l'isomérisation du trans-1-chloro-3,3,3-trifluoropropène en cis-1-chloro-3,3,3-trifluoropropène, et/ou pour limiter ou empêcher la dégradation du trans-1-chloro-3,3,3-trifluoropropène.

2. Utilisation selon la revendication 1, dans laquelle le composé alcène est un butène ou un pentène.

3. Utilisation selon l'une des revendications 1 ou 2, dans laquelle le composé alcène présente :
- une température d'ébullition inférieure ou égale à 100°C, de préférence inférieure ou égale à 75°C, et de maniére plus particulièrement préférée inférieure ou égale à 50°C ; et/ou
- une température de solidification inférieure ou égale à 0°C, de préférence inférieure ou égale à -25°C, et de manière plus particulièrement préférée inférieure ou égale à -50°C.

4. Utilisation selon l'une des revendications 1 à 3, dans laquelle le composé alcène est le 2-méthyl-but-2-ène.

5. Utilisation selon l'une des revendications 1 à 4, dans laquelle le au moins un tamis moléculaire est au moins un adsorbant zéolithique.

6. Utilisation selon la revendication 5, dans laquelle le au moins un adsorbant zéolithique est choisi parmi les zéolithes de type zéolithe A, les zéolithes de type faujasite, les zéolithes de type zéolithe Y, ainsi que les mélanges de celles-ci.

7. Utilisation selon la revendication 5, dans laquelle le au moins un adsorbant zéolithique est choisi parmi la zéolithe 3A, la zéolithe 5A, la zéolithe 13X et les mélanges de celles-ci.

8. Utilisation selon l'une des revendications 1 à 7, dans laquelle on utilise au moins un tamis moléculaire pour adsorber de l'air et au moins un tamis moléculaire pour adsorber de l'eau, de préférence disposés en couches successives dans une cartouche.

9. Procédé de chauffage ou de refroidissement d'un fluide ou d'un corps au moyen d'un circuit de compression de vapeur contenant un fluide de transfert de chaleur, ledit procédé comprenant successivement : l'évaporation du fluide de transfert de chaleur, la compression du fluide de transfert de chaleur, la condensation du fluide de transfert chaleur et la détente du fluide de transfert de chaleur, dans lequel le fluide de transfert de chaleur comprend du trans-1-chloro-3,3,3-trifluoropropène et un composé alcène en C3 à C6, et dans lequel le fluide de transfert de chaleur est mis en contact avec un tamis moléculaire.

10. Procédé de production d'électricité au moyen d'un moteur thermique contenant un fluide de transfert de chaleur, ledit procédé comprenant successivement : l'évaporation du fluide de transfert de chaleur, la détente du fluide de transfert de chaleur dans une turbine permettant de générer de l'électricité, la condensation du fluide de transfert de chaleur et la compression du fluide de transfert de chaleur, dans lequel le fluide de transfert de chaleur comprend du trans-1-chloro-3,3,3-trifluoropropène et un composé alcène en C3 à C6, et dans lequel le fluide de transfert de chaleur est mis en contact avec un tamis moléculaire.

11. Procédé selon la revendication 8 ou 9, dans lequel le fluide de transfert de chaleur atteint une température supérieure ou égale à 100°C, de préférence supérieure ou égale à 150°C, de préférence encore supérieure ou égale à 200°C, de préférence encore supérieure ou égale à 220°C.

12. Installation de transfert de chaleur comprenant un circuit de compression de vapeur contenant un fluide de transfert de chaleur, le fluide de transfert de chaleur comprenant du trans-1-chloro-3,3,3-trifluoropropène et un composé alcène en C3 à C6, et le circuit de compression de vapeur étant pourvu d'un tamis moléculaire.

13. Installation selon la revendication 12, choisie parmi les installations mobiles ou stationnaires de chauffage par pompe à chaleur, de climatisation, de réfrigération, de congélation et les moteurs thermiques.

14. Procédé ou installation selon l'une des revendications 8 à 13, dans lequel ou laquelle le composé alcène est le 2-méthyl-but-2-ène.

15. Procédé ou installation selon l'une des revendications 8 à 14, dans lequel ou laquelle le tamis moléculaire est un adsorbant zéolithique, ou de préférence au moins deux adsorbants zéolithiques disposés en couches.

## Patentansprüche

1. Gemeinsame Verwendung einer C₃- bis C₆-Alkenverbindung mit einer einzigen Doppelbindung und mindestens eines Molsiebs zur Einschränkung oder Verhinderung der Isomerisierung von trans-1-Chlor-3,3,3-trifluorpropen zu cis-1-Chlor-3,3,3-trifluorpropen und/oder zur Einschränkung oder Verhinderung des Abbaus von trans-1-Chlor-3,3,3-trifluorpropen.

2. Verwendung nach Anspruch 1, wobei es sich bei der Alkenverbindung um ein Buten oder ein Penten handelt.

3. Verwendung nach Anspruch 1 oder 2, wobei die Alkenverbindung
- einen Siedepunkt kleiner oder gleich 100 °C, vorzugsweise kleiner oder gleich 75 °C und spezieller bevorzugt kleiner oder gleich 50 °C und/oder
- eine Verfestigungstemperatur kleiner oder gleich 0 °C, vorzugsweise kleiner oder gleich -25°C und spezieller bevorzugt kleiner oder gleich -50 °C
aufweist.

4. Verwendung nach einem der Ansprüche 1 bis 3, wobei es sich bei der Alkenverbindung um 2-Methylbut-2-en handelt.

5. Verwendung nach einem der Ansprüche 1 bis 4, wobei es sich bei dem mindestens einen Molsieb um ein zeolithisches Adsorptionsmittel handelt.

6. Verwendung nach Anspruch 5, wobei das mindestens eine zeolithische Adsorptionsmittel aus Zeolithen vom Typ Zeolith A, Zeolithen vom Faujasit-Typ, Zeolithen vom Typ Zeolith Y sowie Mischungen davon ausgewählt ist.

7. Verwendung nach Anspruch 5, wobei das mindestens eine zeolithische Adsorptionsmittel aus Zeolith 3A, Zeolith 5A, Zeolith 13X und Mischungen davon ausgewählt ist.

8. Verwendung nach einem der Ansprüche 1 bis 7, wobei mindestens ein Molsieb zur Absorption von Luft und mindestens ein Molsieb zur Absorption von Wasser verwendet wird, wobei die Molsiebe vorzugsweise in aufeinanderfolgenden Schichten in einer Kartusche angeordnet sind.

9. Verfahren zum Erwärmen oder Abkühlen eines Fluids oder eines Körpers mittels eines Dampfverdichtungskreislaufs, der ein Wärmeübertragungsfluid enthält, bei dem man nacheinander das Wärmeübertragungsfluid verdampft, das Wärmeübertragungsfluid verdichtet, das Wärmeübertragungsfluid kondensiert und das Wärmeübertragungsfluid entspannt, wobei das Wärmeübertragungsfluid trans-1-Chlor-3,3,3-trifluorpropen und eine C₃- bis C₆-Alkenverbindung umfasst und wobei das Wärmeübertragungsfluid mit einem Molsieb in Kontakt gebracht wird.

10. Verfahren zur Erzeugung von Strom mittels einer Wärmekraftmaschine, der ein Wärmeübertragungsfluid enthält, bei dem man nacheinander das Wärmeübertragungsfluid verdampft, das Wärmeübertragungsfluid in einer Turbine, die die Erzeugung von Strom ermöglicht, entspannt, das Wärmeübertragungsfluid kondensiert und das Wärmeübertragungsfluid verdichtet, wobei das Wärmeübertragungsfluid trans-1-Chlor-3,3,3-trifluorpropen und eine C₃- bis C₆-Alkenverbindung umfasst und wobei das Wärmeübertragungsfluid mit einem Molsieb in Kontakt gebracht wird.

11. Verfahren nach Anspruch 8 oder 9, wobei das Wärmeübertragungsfluid eine Temperatur größer oder gleich 100 °C, vorzugsweise größer oder gleich 150 °C, noch weiter bevorzugt größer oder gleich 200 °C, noch weiter bevorzugt größer oder gleich 220 °C, erreicht.

12. Wärmeübertragungsanlage, umfassend einen Dampfverdichtungskreislauf, der ein Wärmeübertragungsfluid enthält, wobei das Wärmeübertragungsfluid trans-1-Chlor-3,3,3-trifluorpropen und eine C₃- bis C₆-Alkenverbindung umfasst und der Dampfverdichtungskreislauf mit einem Molsieb ausgestattet ist.

13. Anlage nach Anspruch 12, ausgewählt aus mobilen oder stationären Wärmepumpenheizanlagen, Klimaanlagen, Kühlanlagen, Gefrieranlagen und Wärmekraftmaschinen.

14. Verfahren bzw. Vorrichtung nach einem der Ansprüche 8 bis 13, wobei es sich bei der Alkenverbindung um 2-Methylbut-2-en handelt.

15. Verfahren bzw. Vorrichtung nach einem der Ansprüche 8 bis 14, wobei es sich bei dem Molsieb um ein zeolithisches Adsorptionsmittel oder vorzugsweise mindestens zwei in Schichten angeordnete zeolithische Adsorptionsmittel handelt.

## Claims

1. The combined use of a C3 to C6 alkene compound including only one double bond and of at least one molecular sieve, for limiting or preventing the isomerization of trans-1-chloro-3,3,3-trifluoropropene to cis-1-chloro-3,3,3-trifluoropropene, and/or for limiting or preventing the degradation of trans-1-chloro-3,3,3-trifluoropropene.

2. The use as claimed in claim 1, in which the alkene compound is a butene or a pentene.

3. The use as claimed in either of claims 1 and 2, in which the alkene compound has:
- a boiling point of less than or equal to 100°C, preferably less than or equal to 75°C and more particularly preferably less than or equal to 50°C; and/or
- a solidification temperature of less than or equal to 0°C, preferably less than or equal to -25°C and more particularly preferably less than or equal to -50°C.

4. The use as claimed in one of claims 1 to 3, in which the alkene compound is 2-methylbut-2-ene.

5. The use as claimed in one of claims 1 to 4, in which the at least one molecular sieve is at least one zeolitic adsorbent.

6. The use as claimed in claim 5, in which the at least one zeolitic adsorbent is chosen from A-type zeolites, faujasite type zeolites, Y-type zeolites, and mixtures thereof.

7. The use as claimed in claim 5, in which the at least one zeolitic adsorbent is chosen from zeolite 3A, zeolite 5A, zeolite 13X and mixtures thereof.

8. The use as claimed in one of claims 1 to 7, in which at least one molecular sieve for adsorbing air and at least one molecular sieve for adsorbing water are used, preferably arranged in successive layers in a cartridge.

9. A process for heating or cooling a fluid or a body by means of a vapor compression circuit containing a heat transfer fluid, said process comprising in succession: evaporation of the heat transfer fluid, compression of the heat transfer fluid, condensation of the heat transfer fluid and expansion of the heat transfer fluid, in which the heat transfer fluid comprises trans-1-chloro-3,3,3-trifluoropropene and a C3 to C6 alkene compound, and in which the heat transfer fluid is placed in contact with a molecular sieve.

10. A process for producing electricity by means of a heat engine containing a heat transfer fluid, said process comprising in succession: evaporation of the heat transfer fluid, expansion of the heat transfer fluid in an electricity-generating turbine, condensation of the heat transfer fluid and compression of the heat transfer fluid, in which the heat transfer fluid comprises trans-1-chloro-3,3,3-trifluoropropene and a C3 to C6 alkene compound, and in which the heat transfer fluid is placed in contact with a molecular sieve.

11. The process as claimed in claim 8 or 9, in which the heat transfer fluid reaches a temperature of greater than or equal to 100°C, preferably greater than or equal to 150°C, more preferably greater than or equal to 200°C, more preferably greater than or equal to 220°C.

12. A heat transfer facility comprising a vapor compression circuit containing a heat transfer fluid, the heat transfer fluid comprising trans-1-chloro-3,3,3-trifluoropropene and a C3 to C6 alkene compound, the vapor compression circuit being equipped with a molecular sieve.

13. The facility as claimed in claim 12, chosen from mobile or stationary facilities for heat-pump heating, air conditioning, refrigeration or freezing and heat engines.

14. The process or facility as claimed in one of claims 8 to 13, in which the alkene compound is 2-methylbut-2-ene.

15. The process or facility as claimed in one of claims 8 to 14, in which the molecular sieve is a zeolitic adsorbent or, preferably, at least two zeolitic adsorbents arranged in layers.
